(19) Europäisches Patentamt
European Patent Office
Office européen des brevets

(11) Publication number: **0 220 011**
**A2**

(12) # EUROPEAN PATENT APPLICATION

(21) Application number: 86307721.0

(22) Date of filing: 07.10.86

(51) Int. Cl.⁴: A 61 K 31/435

(30) Priority: 12.10.85 GB 8525202
18.12.85 GB 8531197

(43) Date of publication of application:
29.04.87 Bulletin 87/18

(84) Designated Contracting States:
BE CH DE FR GB IT LI LU NL SE

(71) Applicant: BEECHAM GROUP PLC
Beecham House Great West Road
Brentford Middlesex TW8 9BD(GB)

(72) Inventor: Sanger, Gareth John
Beecham Pharmaceuticals Coldharbour Road
The Pinnacles Harlow, Essex CM19 5AD(GB)

(72) Inventor: King, Francis David
Beecham Pharmaceuticals Coldharbour Road
The Pinnacles Harlow, Essex CM19 5AD(GB)

(72) Inventor: Miner, Wesley Dennis
39 Rancorn Road Westbrook
Margate Kent(GB)

(74) Representative: Jones, Pauline et al,
Beecham Pharmaceuticals Patents & Trade Marks Dept.
Great Burgh Yew Tree Bottom Road
Epsom Surrey KT18 5XQ(GB)

(54) **Use of azabicloalkylbenzamides in the treatment of gastrointestinal motility disorders, migraine, emesis, cluster headaches, trigeminal neuralgia and arrhythmia.**

(57) A method of treatment of undesirable actions of 5-HT on the gastro-intestinal tract; migraine, cluster headaches and trigeminal neuralgia, cytotoxic agent or radiation induced nausea and vomiting and/or arrhythmia in mammals, including humans, which method comprises administering an efffective amount of a compound of formula (I) or a pharmaceutically acceptable salt thereof:

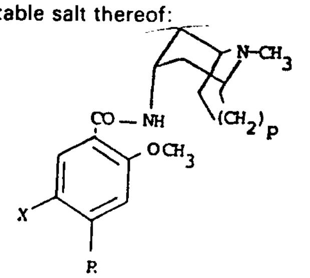

(I)

wherein:

R is amino or $C_{1-7}$ acylamino;

X is chloro, bromo or $C_{1-6}$ alkylthio; and

p is 0 or 1; novel compounds within formula (I) wherein X is bromo or $C_{1-6}$ alkylthio, a process for their preparation and their use as pharmaceuticals.

EP 0 220 011 A2

TITLE MODIFIED
see front page



## NOVEL TREATMENT

This invention relates to a method of treatment of disorders associated with undesirable actions of 5-hydroxytryptamine (5-HT) on the gastro-intestinal tract, migraine, cluster headaches and trigeminal neuralgia, cytotoxic agent or radiation induced nausea and vomiting and/or arrhythmia in mammals, including humans, and to the use of compounds in the preparation of a medicament for the treatment of such conditions. This invention also relates to novel compounds having this pharmacological activity, to a process for their preparation and to their use as pharmaceuticals.

EP-A-13138 and EP-A-67615 disclose classes of substituted azabicyclic compounds which are described as having dopamine antagonist activity, useful in the treatment of disorders relating to impaired gastro-intestinal motility, such as retarded gastric emptying, dyspepsia, flatulence, oesphageal reflux and peptic ulcer. Depending on their balance between peripheral and central action on the nervous system, they may also be used in the treatment of emesis and/or the treatment of disorders of the central nervous system, such as psychosis. The compound 4-amino-5-chloro-2-methoxy-N-(3′α-[8′methyl]-8-azabicyclo-[3.2.1]octyl)benzamide is Compound No.20, described under Example 6, in EP-A-13138.

It has now been discovered that this compound and related compounds are 5-HT M-receptor antagonists and is therefore useful in the treatment of disorders relating to impaired gastro-intestinal motility and also

migraine, cluster headaches and trigeminal neuralgia. They also block the emetic response induced by radiation and cytotoxic agents such as cisplatin, doxorubicin and cyclophosphamide, and are therefore of potential use in the treatment of nausea and vomiting associated with cancer therapy. The compounds are also believed to possess anti-arrhythmic activity.

Accordingly, the present invention provides a method of treatment of undesirable actions of 5-HT on the gastro-intestinal tract; migraine, cluster headaches and trigeminal neuralgia, cytotoxic agent or radiation induced nausea and vomiting and/or arrhythmia in mammals, including humans, which method comprises administering an effective amount of a compound of formula (I) or a pharmaceutically acceptable salt thereof:

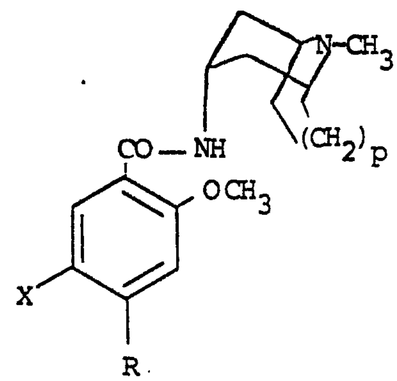

(I)

wherein:

R is amino or $C_{1-7}$ acylamino;
X is chloro, bromo or $C_{1-6}$ alkylthio; and
p is 0 or 1.

Examples of R include amino and $C_{1-6}$ alkanoylamino, such as formylamino, acetylamino, propionylamino, n- and iso-butyrylamino. Often $R_2$ is amino or acetylamino, preferably amino.

Examples of X when $C_{1-6}$ alkylthio include methylthio, ethylthio, n- and iso- propylthio, n-, iso-, sec- and tert- butylthio. Preferably X is methylthio, ethylthio, chloro or bromo, most preferably chloro or bromo.

p may be 0, or 1, preferably 0.

The azabicyclic side chain is in an endo configuration relative to the amide nitrogen in formula (I).

The pharmaceutically acceptable salts of the compound of the formula (I) include acid addition salts with conventional acids such as hydrochloric, hydrobromic, boric, phosphoric, sulphuric acids and pharmaceutically acceptable organic acids such as acetic, tartaric, maleic, citric, succinic, benzoic, ascorbic, methanesulphonic, α-keto glutaric, α-glycerophosphoric, and glucose-1-phosphoric acids.

The pharmaceutically acceptable salts of the compound of the formula (I) are usually acid addition salts with acids such as hydrochloric, hydrobromic, phosphoric, sulphuric, citric, tartaric, lactic and acetic acid. Preferably the acid addition salt is the hydrochloride salt.

Examples of pharmaceutically acceptable salts include quaternary derivatives of the compound of formula (I) include the compounds quaternised by compounds such as $R_{10}$-Z wherein $R_{10}$ is $C_{1-6}$ alkyl, phenyl-$C_{1-6}$ alkyl or $C_{5-7}$ cycloalkyl, and Z is a radical corresponding to an anion of an acid. Suitable examples of $R_{10}$ include methyl, ethyl and n- and iso-propyl; and benzyl and phenethyl. Suitable examples of Z include halide such as chloride, bromide and iodide.

Pharmaceutically acceptable salts also include internal salts such as pharmaceutically acceptable N-oxides.

The compound of the formula (I), its pharmaceutically acceptable salts, (including quaternary derivatives and N-oxides) may also form pharmaceutically acceptable solvates, and these are included wherever a compound of formula (I) or a salt thereof is herein referred to.

The compounds of the formula (I) and salts thereof may be prepared in accordance with the methods described in EP-A-13138 or by analogous methods thereto.

The administration of the compound of formula (I) or a pharmaceutically acceptable salt thereof may be by way of oral or parenteral administration.

An amount effective to treat the disorders hereinbefore described depends on the relative efficacies of the compounds of the invention, the nature and severity of the disorder being treated and the weight of the mammal. However, a unit dose for a 70kg adult will normally contain 0.1 to 50mg for example 0.2 to 20mg, of the compound of formula (I) or a pharmaceutically acceptable salt thereof. Unit doses may be administered once or more than once a day, for example, 2, 3 or 4 times a day, more usually 1 to 3 times a day, that is in the range of approximately 0.001 to 50mg/kg/day, more usually 0.002 to 25mg/kg/day.

No adverse toxicological effects are indicated at any of the aforementioned dosage ranges.

It is preferred that the compound of formula (I) or a pharmaceutically acceptable salt thereof is administered in the form of a unit dose pharmaceutical

composition in which is combined with a pharmaceutically acceptable carrier.

Such compositions are prepared by admixture and are suitably adapted for oral or parenteral administration, and as such may be in the form of tablets, capsules, oral liquid preparations, powders, granules, lozenges, reconstitutable powders, injectable and infusable solutions or suspensions or suppositories. Orally administrable compositions are preferred, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art, for example with an enteric coating.

Suitable fillers for use include cellulose, mannitol, lactose and other similar agents. Suitable disintegrants include starch, polyvinylpolypyrrolidone and starch derivatives such as sodium starch glycollate. Suitable lubricants include, for example, magnesium stearate.

Suitable pharmaceutically acceptable wetting agents include sodium lauryl sulphate. Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending

agents, for example sorbitol, syrup, methyl cellulose, gelatin, hydroxyethylcellulose, carboxymethylcellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

Oral liquid preparations are usually in the form of aqueous or oily suspensions, solutions, emulsions, syrups, or elixirs or are presented as a dry product for reconstitution with water or other suitable vehicle before use. Such liquid preparations may contain conventional additives such as suspending agents, emulsifying agents, non-aqueous vehicles (which may include edible oils), preservatives, and flavouring or colouring agents.

The oral compositions may be prepared by conventional methods of blending, filling or tabletting. Repeated blending operations may be used to distribute the active agent throughout those compositions employing large quantities of fillers. Such operations are, of course, conventional in the art.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the compound in a vehicle and filter sterilising before filling into a

suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure of ethylene oxide before suspending in the sterile vehicle. Advantageously, a surfactant or wetting agent is included in the composition to facilitate uniform distribution of the compound.

The present invention also provides the use of a compound of formula (I) or a pharmaceutically acceptable salt thereof in the preparation of a medicament for use in the treatment of undesirable actions of 5-HT on the gastro-intestinal tract; migraine, cluster headaches and trigeminal neuralgia; cytotoxic agent or radiation induced nausea and vomiting and/or arrhythmia in mammals, including humans. Such treatment may be carried out in the manner as hereinbefore described.

The present invention further provides a pharmaceutical composition for use in the treatment of undesirable actions of 5-HT on the gastro-intestinal tract; migraine, cluster headaches and trigeminal neuralgia, cytotoxic agent or radiation induced nausea and vomiting and/or arrhythmia which comprises an effective amount of a compound of formula (I) or a pharmaceutically acceptable salt thereof and a pharmaceutically acceptable carrier. Such compositions

may be prepared in the manner as hereinbefore described.

The invention also provides novel compounds within formula (I) as hereinbefore defined wherein X is bromo or $C_{1-6}$ alkylthio, and pharmaceutically acceptable salts thereof.

The invention also provides a process for the preparation of a compound of formula (I) wherein X is bromo or $C_{1-6}$ alkylthio, or a pharmaceutically acceptable salt thereof, which process comprises reacting a compound of formula (II):

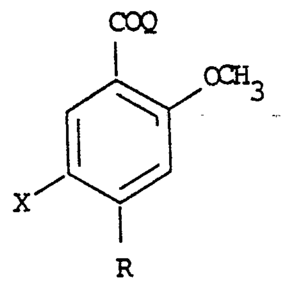

(II)

with a compound of formula (III):

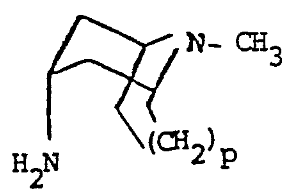

(III)

wherein Q is a leaving group and the remaining variables are as defined in formula (I); and thereafter optionally converting an $R_2$ group to another $R_2$ group, and optionally forming a pharmaceutically acceptable salt of the resultant compound of formula (I).

Examples of leaving groups Q displaceable by a nucleophile include halogen such as chloro and bromo,

hydroxy, carboxylic acyloxy such as $C_{1-4}$ alkanoyloxy or $C_{1-4}$ alkoxycarbonyloxy and activated hydrocarbyloxy such as pentachlorophenoxy.

If a group Q is a halide, then the reaction is preferably carried out at non-extreme temperatures in an inert non-hydroxylic solvent, such as benzene, dichloromethane, toluene, diethyl ether, THF (tetrahydrofuran) or DMF (dimethylformamide). It is also preferably carried out in the presence of an acid acceptor, such as an organic base, in particular a tertiary amine, such as triethylamine, trimethylamine, pyridine or picoline, some of which can also function as the solvent. Alternatively, the acid acceptor can be inorganic, such as calcium carbonate, sodium carbonate or potassium carbonate. Temperatures of $0^{o}$-$100^{o}C$, in particular 10-$80^{o}C$ are suitable.

If a group Q is hydroxy, then the reaction is generally carried out in an inert non-hydroxylic solvent, such as dichloromethane, THF or DMF optionally in the presence of a dehydrating catalyst, such as a carbodiimide, for example dicyclohexylcarbodiimide. The reaction may be carried out at any non-extreme temperature, such as -10 to $100^{o}C$, for example, 0 to $80^{o}C$. Generally, higher reaction temperatures are employed with less active compounds whereas lower temperatures are employed with the more active compounds.

If a group Q is carboxylic acyloxy, then the reaction is preferably carried in substantially the same manner as the reaction when Q is halide. Suitable examples of acyloxy leaving groups include $C_{1-4}$ alkanoyloxy and $C_{1-4}$ alkoxycarbonyloxy, in which case the reaction is preferably carried out in an inert solvent, such as methylene chloride, at a non-extreme temperature for

example ambient temperatures in the presence of an acid acceptor, such as triethylamine.

$C_{1-4}$-alkoxycarbonyloxy leaving groups may be generated in situ by treatment of the corresponding compound wherein Q is hydroxy with a $C_{1-4}$ alkyl chloroformate.

If a group Q is activated hydrocarbyloxy then the reaction is preferably carried out in an inert polar solvent, such as dimethylformamide. It is also preferred that the activated hydrocarbyloxy group is a pentachlorophenyl ester and that the reaction is carried out at ambient temperature.

Pharmaceutically acceptable salts of the compounds of this invention may be formed conventionally.

The salts may be formed for example by reaction of the base compound of formula (I) with a pharmaceutically acceptable organic or inorganic acid.

It will be apparent that compounds of the formula (I) containing an $R_2$ group which is convertible to another R group are useful novel intermediates. A $C_{1-7}$-acylamino substituent is convertible to an amino substituent by deacylation; carried out by treatment with a base, such as an alkali metal hydroxide.

Intermediates of the formula (II) and (III) are known or can be prepared by analogous processes to those used for structurally similar known compounds.

The invention also provides a pharmaceutical composition comprising a compound of formula (I) wherein X is bromo or $C_{1-6}$alkylthio, or a

pharmaceutically acceptable salt thereof, and a pharmaceutically acceptable carrier.

The invention also provides a compound of formula (I) wherein X is bromo or $C_{1-6}$ alkylthio, or a pharmaceutically acceptable salt thereof for use as an active therapeutic substance, in particular for use in the treatment of disorders relating to impaired gastro-intestinal motility and/or emesis and/or migraine, cluster headaches, trigeminal neuralgia and/or radiation or cytotoxic agent induced vomiting and/or arrhythmia.

The following Examples and pharmacological data illustrate the invention.

Compound No. 20 (from EP-A-13138) is (endo)-4-amino-5-chloro-2-methoxy-N-(8-methyl-8-azabicyclo[3.2.1]octyl) benzamide.

Example 1

(endo)-4-Amino-5-bromo-2-methoxy-N-(8-methyl-8-azabicyclo[3,2,1]oct-3-yl) benzamide (E1)

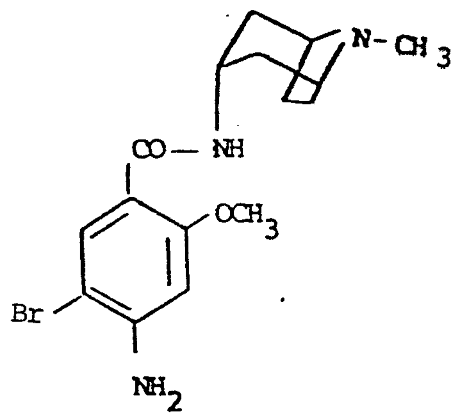

(E1)

To a stirred solution of 4-amino-5-bromo-2-methoxy benzoic acid (0.5g) and triethylamine (0.3ml) in dry, amine-free, DMF (10ml) at $0^{o}C$ was added ethyl chloroformate (0.2ml) and the reaction stirred to room temperature for 1h. A solution of (endo)-8-methyl-8-azabicyclo[3,2,1]octan-3-amine (0.4g) in $CH_2Cl_2$ (5ml) was then added and the whole stirred at room temperature overnight. The solvent was then removed by rotary evaporation *in vacuo* and the residue partitioned between sat. $NaHCO_3$ solution (50ml) and the $CH_2Cl_2$ (100ml). The organic extract was dried ($K_2CO_3$), concentrated and the residue recrystallised from EtOAc/petrol to give the title compound (E1) (0.32g).

m.p. 222-$3^{o}$

$^{1}H$-NMR (79.5MHz, $CDCl_3$)

δ 8.30 (s, 1H)
8.27 (d, 1H)
6.34 (s, 1H)
4.70-3.80 (m, 6H including 3.95, s, 3H)
3.30-3.00 (m, 2H)
2.55-1.50 (m, 11H including 2.32, s, 3H)

Example 2

(endo)-4-Amino-5-methylthio-2-methoxy-N-(8-methyl-8-azabicyclo[3,2,1]oct-3-yl)benzamide (E2)

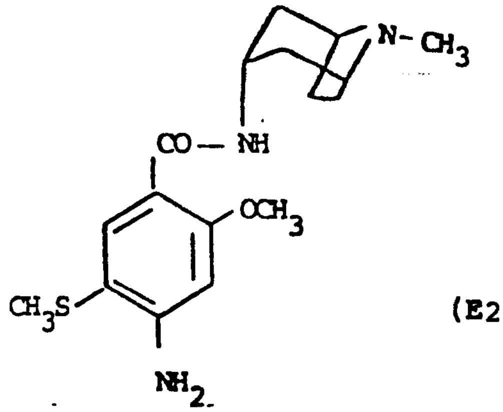

(E2)

Following the procedure outlined in Example 1, 4-amino-5-methylthio-2-methoxybenzoic acid (0.64g) was converted to the title compound (E2) (0.65g).
m.p. 146-7° (EtOAc/petrol)

## Pharmacology

### 1. Intragastric pressure in the rat

Intragastric pressure changes were recorded from fasted conscious and restrained rats using a saline filled catheter inserted into the lumen of the stomach via a permanent gastric fistula. The catheter was connected to a physiological pressure transducer and pressure changes recorded on a hot wire pen recorder. An index of activity was obtained by measuring the average height of pressure waves during 10 minute periods. Values for 4 such periods were obtained during assessment of spontaneous activity prior to dosing and for the 40 minute period following dosing with compound or vehicle. The Student's ''t'' test was applied to the mean values obtained for activity prior to and post treatment. Groups of 10 animals were used for each treatment.

Compound No.20 was active at a dose of 0.13 mg/kg s.c.

### 2. Antagonism of the von Bezold-Jarisch reflex

The compounds were evaluated for antagonism of the von Bezold-Jarisch reflex evoked by 5-HT in the anaesthetised rat according to the following method:

Male rats, 250-350g, were anaesthetised with urethane (1.25g/kg intraperitoneally) and blood pressure and heart rate recorded as described by Fozard J.R. et al., J. Cardiovasc. Pharmacol. 2, 229-245 (1^80). A submaximal dose of 5-HT (usually 6µg/kg) was given repeatedly by the intravenous route and changes in heart rate quantified. Compounds were given intravenously and the concentration required to reduce

the 5-HT-evoked response to 50% of the control response ($ED_{50}$) was then determined.

The result obtained is shown below:

| Compound No. | $ED_{50}$ µg/kg i.v. |
|---|---|
| 20 | 0.8 ± 0.2 |

3. <u>Intraluminal pressure in the Heidenhain pouch of the dog.</u>

Pressure changes were recorded via a saline filled catheter inserted, with airtight closure, into the fistula of a chronic Heidenhain pouch of the previously fasted and lightly restrained conscious dog. The catheter was connected to a physiological pressure transducer and pressure changes recorded on a hot wire pen recorder. Compounds were administered when the motility was in a phase of relatively low activity and the dose range determined which induced an increase in the amplitude of rhythmical contractions for a period of at least 4-5 minutes.

The Compound of Example 1 was active at a dose of 0.01mg/kg i.v.

C

Claims

1. The use of a compound of formula (I) or a pharmaceutically acceptable salt thereof:

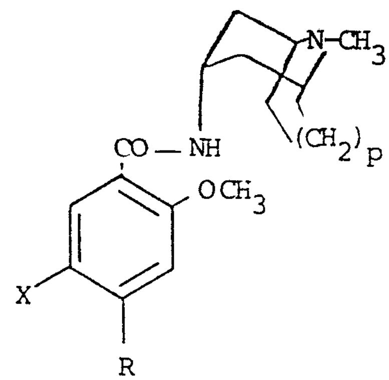

(I)

wherein:

R is amino or $C_{1-7}$ acylamino;

X is chloro, bromo or $C_{1-6}$ alkylthio; and

p is 0 or 1, for the manufacture of a medicament for the treatment of undesirable actions of 5-HT on the gastro-intestinal tract; migraine, cluster headaches and trigeminal neuralgia, cytotoxic agent or radiation induced nausea and vomiting and/or arrhythmia.

2. A use according to claim 1 wherein R is amino.

3. A use according to claim 1 wherein X is chloro, bromo or methylthio.

4. A use according to claim 1 wherein p is 0.

5. A use according to claim 1 wherein the compound of formula (I) is

(endo)-4-amino-5-chloro-2-methoxy-N-(8-methyl-8-azabicyclo(3,2,1)oct-3-yl)benzamide,

(endo)-4-amino-5-bromo-2-methoxy-N-(8-methyl-8-azabicyclo(3,2,1)oct-3-yl)benzamide or

(endo)-4-amino-5-methylthio-2-methoxy-N-(8-methyl-8-azabicyclo(3,2,1)oct-3-yl)benzamide.

6. A compound of formula (I) as defined in claim 1, wherein X is bromo or $C_{1-6}$alkylthio, or a pharmaceutically acceptable salt thereof.

7. (endo)-4-Amino-5-bromo-2-methoxy-N-(8-methyl-8-azabicyclo(3,2,1)oct-3-yl)benzamide or

(endo)-4-amino-5-methylthio-2-methoxy-N-(8-methyl-8-azabicyclo(3,2,1)oct-3-yl)benzamide.

8. A process for the preparation of a compound according to claim 6, which process comprises reacting a compound of formula (II):

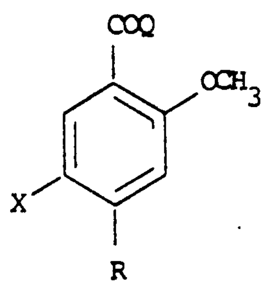

(II)

with a compound of formula (III):

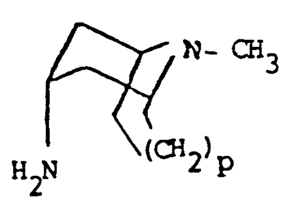

(III)

wherein Q is a leaving group and the remaining variables are as defined in claim 1; and thereafter optionally converting an $R_2$ group to another $R_2$ group, and optionally forming a pharmaceutically acceptable salt of the resultant compound of formula (I).

9. A pharmaceutical composition comprising a compound according to claim 6 and a pharmaceutically acceptable carrier.

10. A compound according to claim 6 for use as an active therapeutic substance.